(19) Europäisches Patentamt
European Patent Office
Office européen des brevets

(11) **EP 2 570 083 A1**

(12) **EUROPEAN PATENT APPLICATION**

(43) Date of publication:
**20.03.2013 Bulletin 2013/12**

(51) Int Cl.:
**A61B 8/08** (2006.01)

(21) Application number: **11181589.0**

(22) Date of filing: **16.09.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Theraclion**
**Malakoff (FR)**

(72) Inventors:
• **Lacoste, Francois**
 **94250 Gentilly (FR)**
• **Yon, Silvain**
 **92220 Bagneux (FR)**
• **Pechoux, Thierry**
 **75019 Paris (FR)**

(74) Representative: **Hepp Wenger Ryffel AG**
 **Friedtalweg 5**
 **9500 Wil (CH)**

(54) **Medical support for a body part**

(57) The present invention relates to a medical support (1) for a body part (2) comprising at least one envelope with an outer wall (3) defining a cavity (4) within said envelope. The outer wall (4) includes at least one contact area (8) to receive a body part from a patient. This at least one contact area (8) is made of a flexible material having an acoustic impedance of from $1.4 \cdot 10^6$ Pa·s·m$^{-1}$ to $1.7 \cdot 10^6$ Pa·s·m$^{-1}$, preferably of $1.56 \cdot 10^6$ Pa·s·m$^{-1}$.

Fig. 1

EP 2 570 083 A1

## Description

This invention relates to a medical support for a body part according to claim 1 and an ultrasound device comprising at least one medical support according to claim 14.

Ultrasound waves of the High Intensity Focused Ultrasound (HIFU) type may be used to destroy a target tissue or a tumor. By application of very high intensity ultrasound waves to a target area, cells are heavily damaged or destroyed. This is mainly caused by generation of heat by the ultrasound waves in the target area. Further, the cavitation effect caused by the ultrasound waves as well as mechanical vibration can cause additional damage to the cells.

Typically, HIFU waves have a frequency in the range of approximately 0.2MHz to 3.5MHz which reach an intensity of 500W/cm$^2$ up to 50kW/cm$^2$ at the focal point of the HIFU sound wave beam.

One major problem for both imaging and treatment application of ultrasound is the movement of the patient or the body part to be imaged or treated. While in imaging applications movement will only result in blurring of the image, the consequences of movement in the case of HIFU treatment may be serious damages to healthy tissue.

Another problem is to bring and hold a body part in a position favorable for the imaging or treatment of a specific area, e.g. a tumor or the like. This is especially a problem when the body part to be imaged or treated may not or only very restrictively be actively moved by the patient. This is specifically the case for imaging or treatment of female breasts.

In the state of the art there are various solutions known to position and hold a female breast in a favorable position during imaging or interstitial treatment.

US 6,304,770 for example describes a breast stabilization device for imaging and invasive medical procedures. The device includes a shell configured to surround a superior portion of a breast when the breast rests on a substantially flat surface. The shell comprises an opening allowing access to the breast for an ultrasound imaging transducer.

US 7,142,631 discloses a mammography device comprising a compressible bolster and a separate x-ray transparent compressible pad. The bolster retains the pad on the imaging surface. A separate stretchable retainer holds a breast and serves as an exposure equalizer. A compression paddle adapted with a bolster and a light transparent panel facilitates proper placement of a breast for compression.

US 7,828,744 describes an assembly for breast immobilization with a cone placed above a breast of a patient lying in a supine position. A vacuum is applied to a multitude of openings inside the cone to hold the breast upright and away of the chest of the patient.

WO 2006/007423 discloses an apparatus and method for treating a breast, e.g. by means of HIFU treatment. This application proposes to provide a treatment table with an opening therethrough for insertion of a breast. The breast is held in a fixed position by means of a special bra or cone shaped membrane. The breast is drawn into the membrane by means of application of a vacuum.

However, all these solutions have several drawbacks. Some require the patient to lie in a prone position for the duration of the treatment, which may be perceived as uncomfortable by the patient. Or they require the application of vacuum to "suck" the breast into position which may cause pain to the patient. Further, the use of vacuum devices requires the use of pumps and tubes, which complicates the procedure and reduces the flexibility of use. Other solutions require squeezing a breast between rigid plates which causes pain and discomfort to the patient, especially in the case of treatments lasting up to an hour.

Further, one big disadvantage of most holding devices is that HIFU waves not absorbed by the target tissue are reflected and/or absorbed at the boundary between the skin and surrounding air or a surface onto which the body part under treatment is lying. These reflections and/or absorptions may cause burns to the skin. Further, even if a coupling liquid is applied between the body part and a surface, HIFU waves may be reflected at the boundary between the surface and the body part which will also cause burns or other heat damage to the skin.

Another approach is taken by the US 6,778,848 which proposes to provide a bath for the immersion of a breast into a liquid. The fixation of the breast is done by one or more inflatable compression pads.

While such a device may effectively reduce the amount of HIFU waves reflected or absorbed by the skin, its use is limited since a patient may only be treated in a prone position and has to lie still for the duration of the treatment. Further, this device is rather bulky and may only be re-used after exchange of the liquid and thorough cleaning of the entire device, which is time consuming.

It is therefore an object of the present invention to provide a support for a body part avoiding the disadvantages of the devices as known in the art and which allows for an easy and comfortable fixation of a body part, especially for a female breast, while reducing the risk of heat damage or burns to the skin caused by reflection or absorption of ultrasound waves. This object is achieved by a medical support as claimed in claim 1.

The medical support for a body part according to the present invention comprises at least one envelope with an outer wall. Said outer wall defines a cavity within said envelope. The outer wall further is provided with at least one contact area sized and shaped to receive a body part from a patient. Said at least one contact area is made of a flexible material having an acoustic impedance of from $1.4 \cdot 10^6$ Pa·s·m$^{-1}$ to $1.7 \cdot 10^6$ Pa·s·m$^{-1}$, preferably of $1.56 \cdot 10^6$ Pa·s·m$^{-1}$.

The outer wall of the envelope has a three dimensional shape enclosing a cavity. The shape of the envelope may be of any suitable form and size, depending on the intended use. The at least one contact area to receive a body part from a patient is preferably unitary with the outer wall, i.e. the outer wall and the at least one contact area are made of a single piece of material. Alternatively, the at least one contact area may be attached to the outer wall. However, such attachment has to be tight and must be able to withstand pressure forces. Hence, the at least one contact area is preferably attached to the outer wall by means of welding or adhesive bonding.

The at least one contact area is preferably made of a flexible material, e.g. a foil or membrane. The material further preferably has an acoustic impedance of from $1.4 \cdot 10^6$ Pa·s·m$^{-1}$ to $1.7 \cdot 10^6$ Pa·s·m$^{-1}$ and more preferably of $1.56 \cdot 10^6$ Pa·s·m$^{-1}$. A material having an acoustic impedance in this range highly reduces the rate of reflection of HIFU waves at a boundary between said material and human skin. Specifically, use of such a material may reduce the rate of reflected HIFU waves compared to other material. As is known in the art, the rate of reflection of sound waves at a boundary between two materials may be calculated using the following formula:

$$ R = \frac{(z_2 - z_1)}{(z_2 + z_1)} $$

where $z_1$ and $z_2$ are the acoustic impedance values of the two different materials at the boundary.

Further, the material of said at least one contact area preferably has a thermal conductance of at least $500\,\mathrm{W \cdot m^{-2} \cdot K^{-1}}$, preferably of at least $1000\,\mathrm{W \cdot m^{-2} \cdot K^{-1}}$. This allows a rapid heat transfer from the skin to the envelope and therefore helps to attenuate the impact of heat generated by the remaining HIFU waves being reflected. Additionally, a high heat conductance also allows the rapid dissipation of heat generated by absorbance of HIFU waves by the material itself.

Preferred materials are for example latex or a material comprising a silicone compound, most preferably MED-4050 obtainable from NuSil Silicone Technology of Carpentera, Florida.

The material preferably has a thickness of less than 0.5mm, more preferably less than 0.25mm, most preferably less than 0.125mm. Using a thin material reduces the absorption rate of HIFU waves from the material itself and thus reduces the heat generation.

Further, providing a thin material further enhances heat dissipation across the material.

Additionally, the at least one contact area is preferably made of a material showing a low absorbance to HIFU waves. More preferably, the at least one contact area is made of a silicone compound and/or latex.

The cavity defined by said outer wall preferably is filled with a fluid, a gel or an oil. The fluid, gel or oil may carry away any heat transferred from the skin through the material of the at least one contact area, therefore providing a cooling effect to the skin. Suitable coupling fluids or gels are known to a person having skill in the art. Further, the cavity may also be filled with an oil, such as castor oil, phenylated silicone oil (such as Dow-Corning® 710 Fluid) or the like.

More preferably, the attenuation coefficient of the fluid or gel for HIFU waves is such, that at least 85%, preferably at least 90% of the HIFU waves which enter the cavity are attenuated when they reach the other side of the envelope. For example, if the envelope has a dimension of 3cm generally in the direction of propagation of the HIFU waves, use of castor oil as fluid in the cavity will result in an attenuation of HIFU waves of 89%.

Preferably, the fluid or gel is an ultrasound coupling liquid, an ultrasound coupling gel or an oil having an acoustic impedance of from $1.4 \cdot 10^6$ Pa·s·m$^{-1}$ to $1.7 \cdot 10^6$ Pa·s·m$^{-1}$ and more preferably of $1.56 \cdot 10^6$ Pa·s·m$^{-1}$.

The contact area is preferably shaped such as to conform to the shape of a specific body part. The contact area is more preferably pre-formed such as to roughly conform to the contour of a body part, e.g. a leg or an arm. Most preferably the contact area is shaped such as to conform to the shape of a female breast. The contact area preferably includes a generally conically shaped recess adapted to receive a breast therein. Preferably, the contact area thereby has a diameter form 5cm to 10cm on the outer wall of the envelope.

The envelope is preferably held in a rigid frame. Alternatively, a rigid frame is arranged within said envelope. In both cases the frame is configured in such a way that at least said at least one contact area remains flexible to a certain degree, such as to conform to the outer shape of the body part. Therefore, the frame imparts rigidity to the envelope with the exception of the at least one contact area. The frame may comprise support walls and/or support rods of a rigid material, preferably a plastic material and/or a metallic material. The frame more preferably imparts a specific shape to the envelope. Further, the rigid frame protects the envelope from overstressing which may lead to bursting of the envelope. Most preferably the frame imparts rigidity to all other areas of the envelope with the exception of the at least one contact area to receive a body part. Alternatively, the frame may also be configured such that other areas of the envelope remain flexible.

Preferably, the medical support comprises means to hold and/or press the body part onto said at least one contact area. This enables to securely attach the body part onto the medical support. Further, the body part may be secured and hold onto the medical support in a defined spatial position, which facilitates positioning an ultrasound transducer, e.g. a high intensity focused ultrasound transducer on the body part. Additionally, different pressure forces may be applied on the body part by varying the compression force exerted by the holding means onto the body part, such as e.g. to compress a human breast for diagnosis or for treatment.

The holding and/or pressing means are preferably in the form of at least a strap, a flexible sheet, a plate and/or a ring. By varying the length of the strap or flexible sheet, it is possible to apply different compression forces onto the body part. If a plate or ring is used, an appropriate holder for said plate or ring is provided, which enables to move said plate or ring in relation to the medical support, thus varying the distance between the medical support and the plate or ring. In the case that a plate is used, it is preferred that the plate includes at least one opening allowing direct access to the body part, such as to enable a direct contact with an ultrasound probe head. The at least one opening may thereby be shaped and sized such as to allow access to a major portion of the body part, while still enabling the application of a force onto the body part, which is sufficient to hold it on the medical support or to exert some compression onto it.

Preferably, the medical support additionally includes means for applying and/or maintaining a pressure force onto said body part. Such means allow to press the body part into the medical support and also to conform to a defined shape, e.g. by providing a shape on the medical support and/or on the pressure force means to which the body part will conform to upon the application of a pressure force. It is also possible to reduce the distance of a target area to be treated to the surface of the body part by compressing the body part. This allows e.g. to reduce the amount of energy needed in a high intensity focused ultrasound treatment, which helps reducing heat damage to healthy tissue between the transducer and the target area.

The medical support preferably includes at least one connection means adapted to removably connect the envelope to a fluid circuit. This allows providing a continuous flow of a fluid, preferably an ultrasound coupling liquid, through the envelope. Most preferably, the envelope may be coupled to a liquid cycle comprising a cooling means, such that the fluid is cycled from the cooling means to the cavity and back, thereby providing a cooling effect to the body part. This helps to reduce heat induced damage to healthy tissue, e.g. during a high intensity focused ultrasound treatment.

Preferably, the medical support includes at least one support arm being movable and/or rotatable in relation to the patient and/or the body part. This enables to variably position the medical support in relation to the patient and/or the body part. The support arm is preferably mountable onto a rigid structure, such as a stretcher or the like. Alternatively, the support arm is mounted on a pedestal, most preferably comprising wheels or the like enabling to move the arm around in a room. The support arm further preferably comprises means to vary the distance of the medical support from the floor or the rigid structure. Additionally, the support arm more preferably also comprises means to vary at least one angle of the medical support in relation to the patient and/or the body part.

The medical support preferably includes connection means to removably connect said medical support to an ultrasound device, preferably of the high intensity focused ultrasound type, allowing to couple the medical support and the transducer in a predefined and temporarily invariable position to one another. More preferably, the connection means are configured such that the relative position of the transducer may be altered by an operator prior to a diagnosis or treatment, e.g. such as to "aim" the transducer towards a target area of the body part.

Another objective of the present invention is to provide an ultrasound device which allows for an easy and comfortable fixation of a body part reducing the incidence of heat induced damages to healthy tissue. This objective is achieved with a device according to claim 13.

The ultrasound device comprises at least one medical support according to the present invention. This allows placing a body part on an envelope having a contact area which is adapted to receive said body part and which has a favourable acoustic impedance reducing the risk of heat damage or burns to the skin caused by reflection or absorption of ultrasound waves.

Further details and embodiments of the present invention will become apparent from the following description of examples and figures:

Fig. 1:    Shows a section through an embodiment of the medical support of the present invention;

Fig. 2:    is a representation of a section of a second embodiment of the medical support;

Fig. 3:    shows a third embodiment of a medical support with a holding and pressing means;

Fig. 4:    is a representation of a medical support with an alternative holding and pressing means;

Fig. 5:    shows a further alternative of a holding and pressing means of a medical support;

Fig. 6:    represents an alternative embodiment of the medical support shown on figure 5; and

Fig. 7:    shows a further embodiment of a medical support with a support arm.

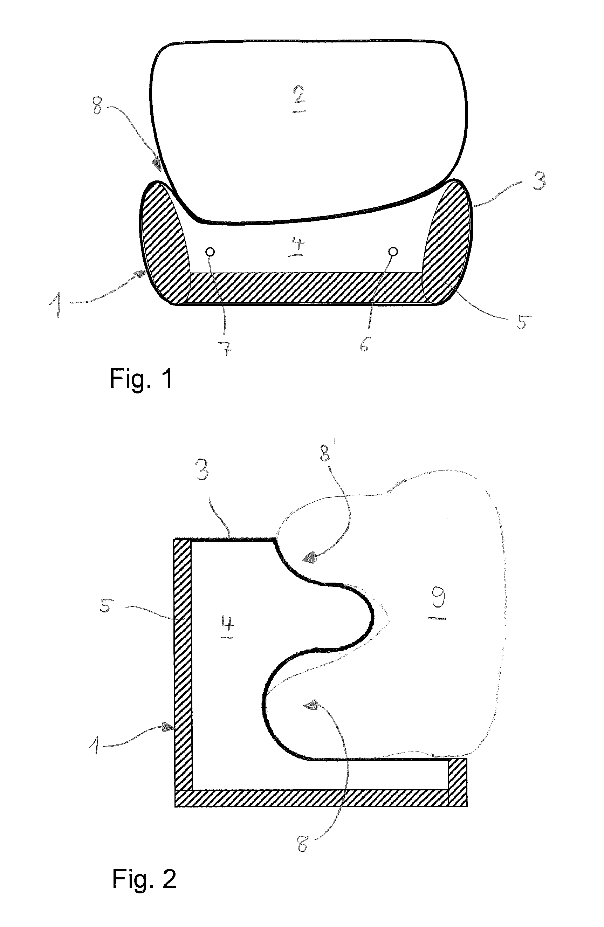

Figure 1 shows a section through an exemplary embodiment of a medical support 1 according to the present invention. The medical support 1 comprises an envelope with an outer wall 3. The outer wall 3 encloses a rigid frame 5 and defines a cavity 4 which is filled with an ultrasound coupling liquid, an ultrasound coupling gel, an oil or with water. The cavity 4 may be brought into connection with a fluid circuit, such as a coupling liquid circuit, by means of an inlet 6 and an outlet 7. Warm coupling liquid may be pumped out of the outlet 7 and fresh, preferably cooled coupling liquid may be provided through inlet 6. The use of a fluid circuit, especially of a fluid circuit comprising an active cooling of the fluid, additionally helps in reducing the risk of heat damages or burns caused by e.g. a high intensity focused ultrasound treatment. Inlet 6 and outlet 7 may be provided in any suitable form, such as e.g. a valve or the like. The rigid frame 5 imparts some rigidity to the outer wall 3 of the envelope. However, a contact area 8 adapted to receive a body part 2 remains flexible, as frame 5 does not support said contact area 8. The acoustic impedance of the outer wall 3 of the contact area 8 is from $1.4 \cdot 10^6$ Pa·s·m$^{-1}$ to $1.7 \cdot 10^6$ Pa·s·m$^{-1}$, preferably of $1.56 \cdot 10^6$ Pa·s·m$^{-1}$. The acoustic impedance of the coupling liquid, the ultrasound coupling gel or the oil filled into cavity 4 preferably has the same or substantially the same acoustic impedance as the contact area 8. The outer wall 3 may be made of different materials for the contact area 8 and the parts of the outer wall 3 which are juxtaposed to the rigid frame 5, but preferably the outer wall 3 is made entirely of the same material. The contact area 8 may be pre-shaped to easily accommodate a specific body part, such as a human breast, a leg, an arm, etc. The medical support 1 is preferably of rectangular shape with a dimension of 12cm x 15cm. The height of the medical support 1 preferably is 2cm.

Figure 2 is a representation of a section of another embodiment of a medical support 1. In this embodiment, the outer wall 3 of the envelope is shaped to accommodate two human breasts with a patient 9 lying on the side. The outer wall 3 of the envelope is thereby shaped in such a way as to provide two contact areas 8, 8' adapted to receive the breasts of the patient 9. The rigid frame 5 imparts some rigidity to the medical support 1 and the cavity 4 is filled with an ultrasound coupling liquid, an ultrasound coupling gel, an oil or water. Around the two contact areas 8 and 8', the outer wall 3 is flexible, such as to allow a comfortable accommodation of the breasts.

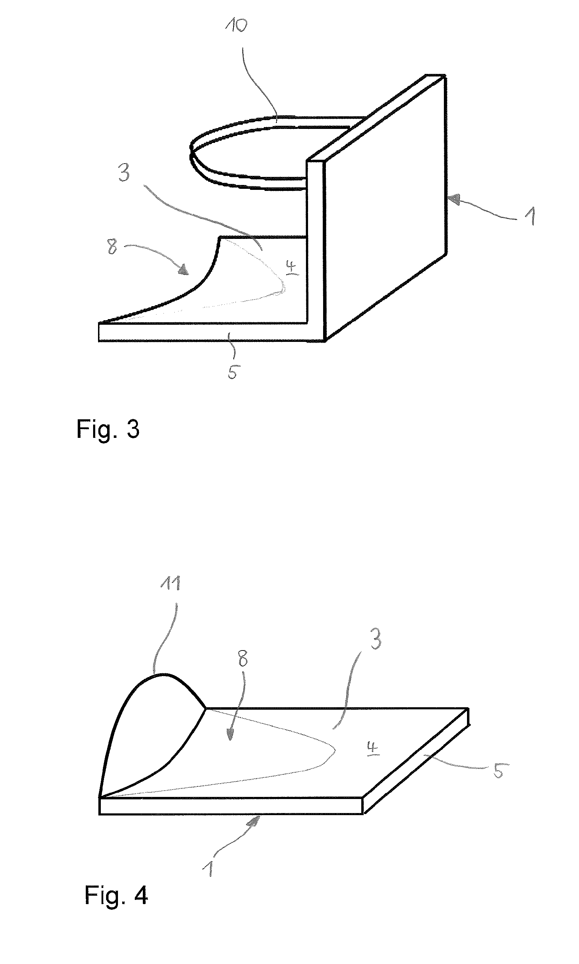

Figure 3 shows an embodiment of the medical support 1 with means to hold and/or press a body part onto the medical support 1. The means comprise a ring 10, which is movable in relation to the contact area 8 adapted to receive the body part. The ring 10 is thereby movable in such a way as to vary the distance between the contact area 8 and the ring 10. By moving the ring 10 towards the contact area 8, a body part, such as a human breast, may be held tightly onto medical support 1. It is also possible to apply a pressure force onto the body part by further moving the ring 10 towards the contact area 8. The medical support 1 comprises means to move the ring 10 and to secure it at a specific position in relation to the contact area 8 (means not shown). These means may be in the form of guide rails or in any other suitable form. The ring 10 may be secured at a specific position e.g. by means of a set screw or the like. As in the embodiments shown on figures 1 and 2, the medical support 1 comprises an envelope with an outer wall 3 defining a cavity 4 which is filled with an ultrasound coupling liquid, an ultrasound coupling gel, an oil or water.

A medical support with an alternative embodiment of a holding and/or pressing means is shown in figure 4. In this embodiment, the holding and/or pressing means is provided in the form of a flexible strap 11, preferably made of an elastomeric material. A body part may be attached to and held onto the medical support 1 by laying the body part onto the contact area 8 and strapping the flexible strap 11 around the body part. A pressure force may be applied onto the body part by more tightly strapping the flexible strap 11 around the body part. Such as to be adaptable to varying body part sizes and to enable a more or less tight strapping, the flexible strap 11 may be provided with means allowing to vary its length over the body part. These means may be provided in the form of a buckle, a ratchet mechanism or the like.

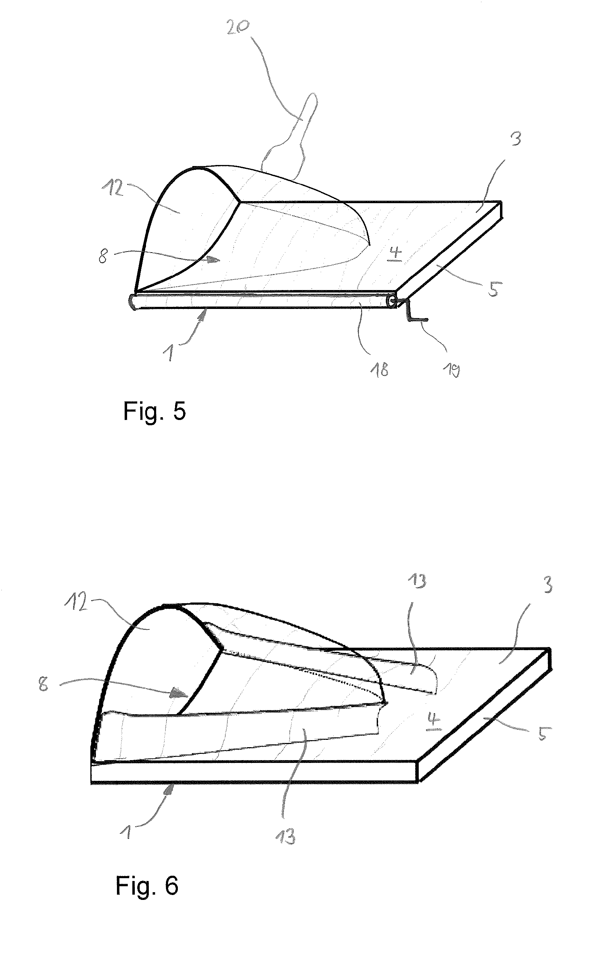

A further alternative embodiment of a holding and/or pressing means for a medical support 1 is shown on figure 5. In this embodiment, the body part is held onto the contact area 8 by means of a flexible foil 12 which is applied over the body part and the outer wall 3 of the envelope. The medical support 1 may comprise at least one roll 18 onto which spare foil 12 is wrapped. The roll 18 preferably comprises means to prevent further unrolling of the foil 12, such as to enable the tightening of the foil 12 onto the body part. Further, the roll also includes an actuation means, such as crank handle 19, to allow an operator to unroll or roll up the foil 12 onto roll 18. The foil 12 is made of material which is translucent for ultrasound waves, like silicon or latex. Preferably, the foil has a thickness of less than 0.1 mm, most preferably 0.05 mm. While the body part is being held onto the medical support 1 by means of the foil 12, HIFU treatment is applied to the body part through the foil 12 by means of a HIFU probe head 20.

In a preferred variant of this embodiment, the medical support 1 includes two rolls 18 mounted on opposite sides of the envelope, allowing to unroll unused foil 12 from one roll 18 and to roll up the used foil 12 onto the other roll 18. Using two rolls 18, the foil 12 may be tightened on the body part by actuating the respective crank handles 19 of both rolls in different directions, thus exerting a tension force on the foil 12. As shown on figure 6, the embodiment of the medical support 1 as shown on figure 5 may further be provided with rigid baffles 13 flanking the contact area 8 adapted to receive the body part. By using such baffles 13, it is possible to impart a specific three dimensional shape to the body part once it is placed on the contact area 8 and a pressure is applied by means of foil 12. The baffles 13 additionally hinder any movement of the body part impaired by the moving around of a probe head.

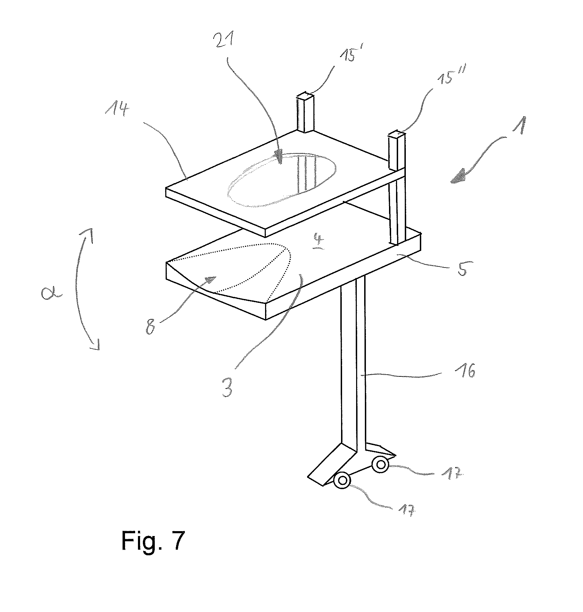

Figure 7 shows one example of a medical support 1 comprising a support arm 16. In this embodiment, the support arm 16 is configured as ground stand, including wheels 17 allowing to move the medical support 1 around on a floor, e.g. of an examination room. The support arm may also be attached to the patient support or bolted to the floor. The holding and/or pressing means are provided in the form of a movable plate 14. The movable plate 14 is configured such as to be movable about columns 15',15" towards or away of the contact area 8 adapted to receive the body part. This allows varying the distance between the contact area 8 and the plate 14 such as to fixate the body part between the contact area 8 and the plate 14. In the embodiment as shown, the columns 15',15" are fixed to the rigid frame 5, so they may extend above the level of plate 14. In an alternative embodiment, the columns 15',15" are fixed to plate 14 and slide relative to the rigid frame 5. This has the advantage that the top of the medical support 1 is free from the columns 15',15", thus allowing easier positioning of the HIFU treatment head. Additionally, by further moving the plate 14 towards the contact area 8, it is possible to apply a compression force onto the body part. The plate 14 preferably comprises means to hold it at a specific distance to the contact area 8, such as a set screw or the like. The plate 14 additionally comprises an opening 21, such as to allow access to the body part by an ultrasound probe head. The opening 21 is shaped and dimensioned in such a way as to allow good access to a major portion of the body part by a probe head, while still enabling the application of a sufficient holding and/or compression force onto the body part. The opening may be open ("U-shaped") or closed at its distal end by a rigid edge or by a flexible strap 11 such as described above. Further, means to vary the inclination angle $\alpha$ of the medical support 1 in relation to the support arm 16 are provided, such as to allow to further vary the position of the medical support 1 relative to the patient, such as to enable a favourable positioning of the medical support 1 to the body part. Additionally, means to slide the medical support 1 along the length of the support arm 16 may also be provided.

As will be evident for a person skilled in the art, any combination of the different features of the medical support 1 other than those described above may also be possible. It is e.g. specifically possible to include an inlet 6 and an outlet 7 to the envelopes of any of the medical supports 1 shown in the figures, such as to enable their connection with a fluid cycle.

## Claims

1. Medical support (1) for a body part (2) comprising at least one envelope with an outer wall (3) defining a cavity (4) within said envelope, **characterized in that** said outer wall (3) includes at least one contact area (8) sized and shaped to receive a body part from a patient, wherein said at least one contact area (8) is made of a flexible material having an acoustic impedance of from $1.4 \cdot 10^6$ Pa·s·m$^{-1}$ to $1.7 \cdot 10^6$ Pa·s·m$^{-1}$, preferably of $1.56 \cdot 10^6$ Pa·s·m$^{-1}$.

2. Medical support (1) according to claim 1, **characterized in that** said material of said at least one contact area (8) has a thermal conductance of at least 500 W·m$^{-2}$·K$^{-1}$, preferably of at least 1000 W·m$^{-2}$·K$^{-1}$.

3. Medical support (1) according to any of claims 1 to 2, **characterized in that** said material of said at least one contact area (8) has a thickness of less than 0.5mm, more preferably less than 0.25mm, most preferably less than 0.125mm.

4. Medical support (1) according to any of claims 1 to 3, **characterized in that** said cavity (4) is filled with a liquid or gel, preferably with an ultrasound coupling liquid, an ultrasound coupling gel or an oil.

5. Medical support (1) according to claim 4, **characterized in that** said liquid or gel has an acoustic impedance of from $1.4 \cdot 10^6$ Pa·s·m$^{-1}$ to $1.7 \cdot 10^6$ Pa·s·m$^{-1}$. preferably of $1.56 \cdot 10^6$ Pa·s·m$^{-1}$.

6. Medical support (1) according to any of claims 1 to 5, **characterized in that** said at least one contact area (8) to receive a body part is shaped such as to conform to the shape of a specific body part (2), preferably to the shape of a female breast.

7. Medical support (1) according to any of claims 1 to 6, **characterized in that** said envelope is hold in a rigid frame (5) or a rigid frame (5) is arranged within said envelope in such a way that at least said at least one contact area (8) remains flexible while the frame (5) imparts rigidity to at least one other area of the envelope.

8. Medical support (1) according to any of claims 1 to 7, **characterized in that** said medical support (1) comprises means (10;11;12;14) to hold and/or press the body part onto said at least one contact area (8).

9. Medical support (1) according to claim 8, **characterized in that** said means (10;11;12;14) to hold and/or press the body part onto said at least one contact area (8) comprise at least a strap (11), a flexible sheet (12), a plate (14) and/or a ring (10).

10. Medical support (1) according to any of claims 8 or 9, **characterized in that** said means (10;11;12;14) to hold and/or press the body part onto said at least one contact area (8) additionally include means for applying and/or maintaining a pressure force onto said body part (2).

11. Medical support (1) according to any of claims 1 to 10, **characterized in that** said envelope includes at least one connection means (6;7) adapted to removably connect the envelope to a fluid circuit.

12. Medical support (1) according to any of claims 1 to 11, **characterized in that** said support means includes at least one support arm (16) being movable and/or rotatable in relation to the patient and/or the body part.

13. Medical support (1) according to any of claims 1 to 12, **characterized in that** said medical support (1) includes connection means to removably connect said medical support to an ultrasound device, preferably of the high intensity focused ultrasound type.

14. An ultrasound device comprising at least one medical support (1) according to any of claims 1 to 13.

Fig. 1

Fig. 2

Fig. 3

Fig. 4

Fig. 5

Fig. 6

Fig. 7

**EP 2 570 083 A1**

Europäisches
Patentamt
European
Patent Office
Office européen
des brevets

# EUROPEAN SEARCH REPORT

Application Number

EP 11 18 1589

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | EP 2 277 449 A1 (SIEMENS SCHWEIZ AG [CH]) 26 January 2011 (2011-01-26)<br>* paragraph [0001] *<br>* paragraph [0007] *<br>* paragraph [0012] *<br>* paragraph [0015] *<br>* figures 1, 2 *<br>----- | 1-6,<br>11-14<br>7-10 | INV.<br>A61B8/08 |
| Y | DE 197 45 400 C1 (SIEMENS AG [DE]) 15 April 1999 (1999-04-15)<br>* abstract; figure 1 *<br>* column 1,, line 5 - line 35 *<br>* column 2, line 16 - line 32 *<br>* column 2, line 63 - column 3, line 26 *<br>* column 4, line 46 - column 5, line 24 *<br>----- | 7-10 | |
| A | US 2005/113692 A1 (HE SHENXU [CN] ET AL) 26 May 2005 (2005-05-26)<br>* abstract; figure 4 *<br>----- | 1-14 | |
| | | | TECHNICAL FIELDS SEARCHED (IPC)<br><br>A61B<br>A61N |

The present search report has been drawn up for all claims

| Place of search | Date of completion of the search | Examiner |
|---|---|---|
| Munich | 26 January 2012 | Sonntag, Anne |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 570 083 A1**

### ANNEX TO THE EUROPEAN SEARCH REPORT
### ON EUROPEAN PATENT APPLICATION NO.

EP 11 18 1589

This annex lists the patent family members relating to the patent documents cited in the above-mentioned European search report.
The members are as contained in the European Patent Office EDP file on
The European Patent Office is in no way liable for these particulars which are merely given for the purpose of information.

26-01-2012

| Patent document cited in search report | | | Publication date | Patent family member(s) | | | Publication date |
|---|---|---|---|---|---|---|---|
| EP 2277449 | | A1 | 26-01-2011 | EP | 2277449 | A1 | 26-01-2011 |
| | | | | WO | 2011009740 | A1 | 27-01-2011 |
| DE 19745400 | | C1 | 15-04-1999 | DE | 19745400 | C1 | 15-04-1999 |
| | | | | JP | 4138968 | B2 | 27-08-2008 |
| | | | | JP | 11192231 | A | 21-07-1999 |
| | | | | US | 6778848 | B1 | 17-08-2004 |
| US 2005113692 | | A1 | 26-05-2005 | CN | 1342501 | A | 03-04-2002 |
| | | | | DE | 10297422 | T5 | 04-11-2004 |
| | | | | GB | 2399019 | A | 08-09-2004 |
| | | | | JP | 2005507749 | A | 24-03-2005 |
| | | | | US | 2005113692 | A1 | 26-05-2005 |
| | | | | WO | 03039674 | A1 | 15-05-2003 |

EPO FORM P0459

For more details about this annex : see Official Journal of the European Patent Office, No. 12/82

**13**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6304770 B
- US 7142631 B
- US 7828744 B
- WO 2006007423 A
- US 6778848 B